Europäisches Patentamt
European Patent Office
Office européen des brevets

(19)

(11) Veröffentlichungsnummer: **0 259 264 A1**

(12)

# EUROPÄISCHE PATENTANMELDUNG

(21) Anmeldenummer: 87810490.0

(22) Anmeldetag: 28.08.87

(51) Int. Cl.4: **C 07 D 209/48**
A 01 N 43/38, C 07 C 149/42,
C 07 C 93/14

(30) Priorität: 03.09.86 CH 3537/86

(43) Veröffentlichungstag der Anmeldung:
09.03.88 Patentblatt 88/10

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

(72) Erfinder: Böhner, Beat, Dr.
Hügelweg 3
CH-4102 Binningen (CH)

Moser, Hans, Dr.
Maispracherstrasse 12
CH-4312 Magden (CH)

Pissiotas, Georg, Dr.
Am Sonnenrain 71
D-7850 Lörrach (DE)

(54) **Neue 4-Methylphthalimide.**

(57) Die Erfindung betrifft neue 4-Methyltetrahydrophthalimide der Formel I

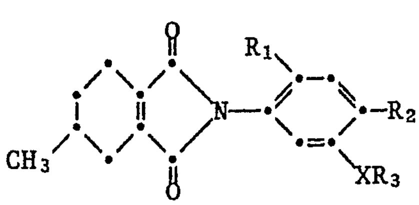

(I) ,

in welcher die Substituenten $R_1$, $R_2$, $R_3$ und X die im Text erläuterte Bedeutung haben, Verfahren zu deren Herstellung sowie neue selektiv-herbizide und pflanzenwachstumsregulatorische Mittel enthaltend Verbindungen der Formel I.

EP 0 259 264 A1

**Beschreibung**

Neue 4-Methylphthalimide

Die vorliegende Erfindung betrifft neue Derivate des N-Phenyltetrahydrophthalimids mit herbizider und pflanzenwuchsregulierender Wirkung, agrochemische Mittel, welche diese Substanzen als Wirkstoffe enthalten, die Verwendung der neuen N-Phenyltetrahydrophthalimide zur selektiven Bekämpfung von Unkräutern oder zur Regulierung von Pflanzenwuchs sowie Verfahren zur Herstellung dieser neuen Verbindungen. Ferner betrifft die Erfindung auch neue Zwischenprodukte, welche zur Herstellung der neuen Wirkstoffe dienen.

Aus der EP-A 61741 sind herbizid wirksame N-Phenyltetrahydrophthalsäureimide bekannt geworden. Diese unter anderem im Phenylimidteil 2,4-dihalogen-5-alkoxy substituierten Verbindungen weisen jedoch im Tetrahydrophthalsäurerest keinen Methylsubstituenten auf. Als Einzelverbindung sei das N[4-Chlor-2-fluor-5-propoxy-phenyl]tetrahydrophthalsäureimid genannt. Die aus dem Stand der Technik bekannten Phthalimide, zeigen bei an sich guter herbizider Wirkung geringe Selektivität.

Es wurde nun überraschenderweise gefunden, dass Verbindungen der Formel I bei guter Selektivität und Kulturpflanzenverträglichkeit herbizid hoch wirksam sind.

Die neuen N-Phenyltetrahydrophthalimid-Derivate entsprechen der Formel I

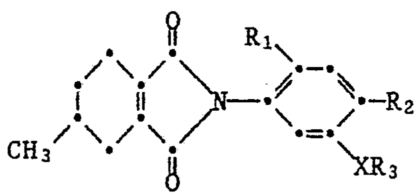

(I) ,

worin

$R_1$ Wasserstoff oder Fluor,

$R_2$ Halogen,

X O oder S,

$R_3$ $C_3$-$C_{10}$-Alkenyl; $C_5$-$C_6$-Cycloalkyl; $c_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;

bedeutet.

Die 4-Methyl-tetrahydrophthalimide der Formel I sind an dem die Methylgruppe tragenden Ringkohlenstoffatom $C_4$ unsymmetrisch substituiert und können somit sowohl in der R als auch in der S-Konfiguration (Formel R-I bzw. S-I) vorliegen.

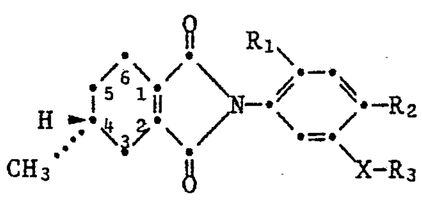

(R-I)

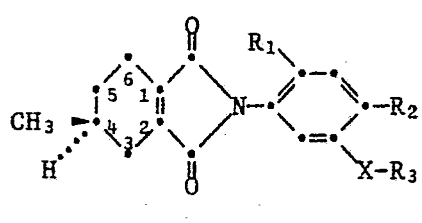

(S-I)

Die Erfindung umfasst beide enantiomeren Formen sowie beliebige Mischungen derselben und das Racemat.

$C_3$-$C_{10}$-Alkenyl sind die einfach, bzw. ab $c_4$ auch mehrfach ungesättigten, cis-trans- wie auch konstitutionsisomeren Radikale; wie etwa das Allyl-, das But-2-en-1-yl- oder das 2-Methyl-prop-2-enyl-radikal.

$C_5$-$C_6$-Cycloalkenyl sind Cyclopentenyl, Cyclohexenyl.

$C_5$-$C_6$-Cycloalkyl sind Cyclopentyl und Cyclohexyl.

$C_3$-$C_{10}$-Alkinyl sind die einfach, bzw. ab $C_4$ auch mehrfach ungesättigten Radikale; wie etwa das Propargyl- oder das But-2-inyl-radikal.

$C_1$-$C_{10}$-Alkyl ist Methyl, Ethyl, Propyl, iso-Propyl, n-Butyl, iso-Buty, tert-Butyl, sek-Butyl, sowie die isomeren Pentyle, wie n-Pentyl, 1-Pentyl (1-Ethyl-propyl), t-Pentyl (1,1-Dimethylpropyl) und die isomeren Hexyl-, Heptyl-, Octyl-, Nonyl- und Decylradikale.

Halogen ist Fluor, Chlor, Brom und Jod, vorzugsweise Fluor, Chlor und Brom.

In den weiteren Substituenten, welche sich durch die Kombination einzelner Teilelemente ergeben, haben

die Teilelemente die im Rahmen der Definitionsbreite gewählte Bedeutung und können durch Kombination von Einzelelementen aus vorstehender Aufzählung frei gewählt werden. Diese Aufzählung bedeutet auch in diesen Fällen keine Einschränkung der Erfindung; sie haben lediglich illustrierenden Charakter.

Bevorzugt sind Verbindungen der Formel I,
worin
$R_1$ Wasserstoff oder Fluor,
$R_2$ Fluor, Chlor oder Brom
X O oder S,
$R_3$ $C_3$-$C_5$-Alkenyl; $C_3$-$C_5$-Alkinyl; Cyclopentyl; Cyclohexenyl; $C_5$-$C_6$-Cycloalkyl; oder $C_1$-$C_6$-Alkyl welches gewünschtenfalls einfach substituiert ist durch $C_1$-$C_4$-Alkoxy, $C_1$-$C_4$-Alkoxycarbonyl, $C_1$-$C_4$-Alkylthio, $C_1$-$C_4$-Alkylthiocarbonyl, Cyano oder ein- oder mehrfach gleich oder verschieden substituiert ist durch Fluor, Chlor oder Brom;
bedeutet.

Unter den als bevorzugt herausgestellten Verbindungen der Formel I sind als einzelne Gruppen zu nennen:

Gruppe A

Verbindungen der Formel I, worin $R_3$ Methyl, Ethyl, Isopropyl, n-Propyl, n-Butyl, sek-Butyl, iso-Butyl, tert.-Butyl, n-Pentyl, iso-Pentyl oder tert.-Pentyl bedeutet.

Gruppe B

Verbindungen der Formel I, worin $R_3$ Allyl, 2-Methylpropenyl oder But-2-en-1-yl bedeutet.

Gruppe C

Verbindungen der Formel I, worin $R_3$ Propargyl oder But-2-inyl bedeutet.

Gruppe D

Verbindungen der Formel I, worin $R_3$ Cyclopentyl, Cyclohexyl, Cyclopentenyl oder Cyclohexenyl bedeutet.

Gruppe E

Verbindungen der Formel I, worin $R_3$ $C_1$-$C_3$-Alkoxycarbonylmethyl oder $C_1$-$C_3$-Alkoxycarbonylethyl bedeutet.

Gruppe F

Verbindungen der Formel I, worin X Sauerstoff bedeutet.

Gruppe G

Verbindungen der Formel I, worin X Schwefel bedeutet.

Gruppe H

Verbindungen der Formel I, worin $R_1$ Wasserstoff und $R_2$ Chlor oder Brom bedeutet.

Gruppe I

Verbindungen der Formel I, worin $R_1$ Fluor und $R_2$ Chlor oder Brom bedeutet.

Als besonders bevorzugte Einzelverbindungen sind zu nennen:
N-(4-Chlor-2-fluor-5-isopropyloxyphenyl)-4-methyl-3,4,5,6-tetrahydrophthalimid,
N-(4-Chlor-2-fluor-5-methoxyphenyl)-4-methyl-3,4,5,6-tetrahydrophthalimid,
N-[4-Chlor-2-fluor-5-(methoxycarbonylmethyl)-phenyl]-4-methyl-3,4,5,6-tetrahydrophthalimid,
N-(4-Chlor-2-fluor-5-isobutyloxy-phenyl)-4-methyl-3,4,5,6-tetrahydrophthalimid,
N-(4-Chlor-2-fluor-5-n-butyloxy-phenyl)-4-methyl-3,4,5,6-tetrahydrophthalimid,
N-(4-Chlor-5-ethoxy-2-fluor-phenyl)-4-methyl-3,4,5,6-tetrahydrophthalimid.

Die Verbindungen der Formel I werden erfindungsgemäss hergestellt,

a) indem man 4-Methyl-3,4,5,6-tetrahydrophthalsäureanhydrid II mit einem Anilin der Formel III,

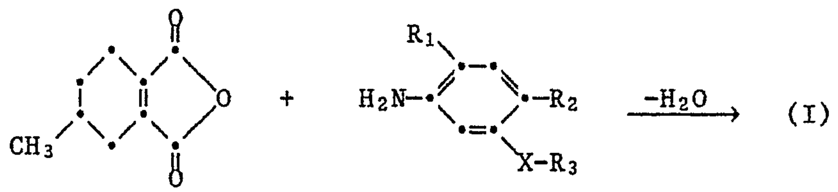


(II) (III)

worin $R_1$, $R_2$, $R_3$ und X die unter Formel I gegebene Bedeutung haben, kondensiert oder

b) in dem man ein Phenol oder Thiophenol der Formel IV mit einer Verbindung der Formel V umsetzt,

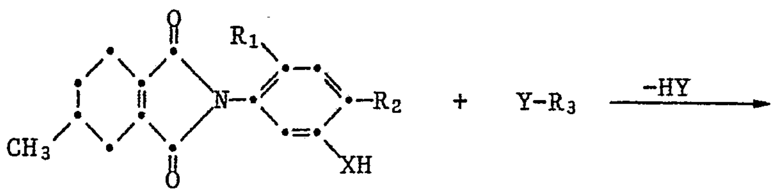


(I)

(IV) (V)

worin $R_1$, $R_2$, $R_3$ und X die unter Formel I gegebene Bedeutung haben und Y für eine unter den Reaktionsbedingungen austauschbare Gruppe, wie Halogen, vorzugsweise Chlor, Brom oder Jod oder für einen gewünschtenfalls im Phenylkern alkylierten oder halogenierten Phenylsulfonylrest oder für ein Radikal der Formel $R_3OSO_2$-O steht.

Mit Vorteil führt man die obigen Kondensationsreaktionen in einem inerten organischen Lösungsmittel aus. Die Reaktionstemperatur liegt im allgemeinen zwischen der Raumtemperatur und der Siedetemperatur des Reaktionsgemisches, vorzugsweise wird die Reaktionsmischung zum Rückfluss erhitzt. Die Kondensationsreaktion a) kann durch Zusatz von Kondensationskatalysatoren und Entfernung des entstehenden Reaktionsproduktes Wasser beschleunigt werden. Eine gleiche Wirkung erzielt man durch Zusatz von wasserentziehenden Mitteln, wie beispielsweise Schwefelsäure.

Als Katalysatoren kommen insbesondere dann in Betracht, wenn ein aprotisches Lösungsmittel verwendet wird: p-Toluolsulfonsäure, Benzoesäure, 4-Dimethylaminopyridin, Schwefelsäure, Chlorwasserstoff oder Naphthalinsulfonsäure. Reaktionsführungen gemäss dem oben genannten Typ werden bei der Darstellung von Carbonsäure-Derivaten üblicherweise angewendet. Sie entsprechen der allgemein üblichen Laboratoriumspraxis.

Im Fall der Reaktion b) ist es vorteilhaft unter Basenzusatz zu arbeiten. Geeignete Basen sind u.a. Natrium-, Kalium- und Calciumhydroxid, Alkali- und Erdalkalicarbonate, Amine, wie etwa Triethylamin oder Heterocyclen, wie Pyridin, DABCO etc. Die Reaktion kann auch vorteilhaft unter Phasentransfer-Bedingungen in Zweiphasensystemen durchgeführt werden. Derartige Reaktionen sind dem Fachmann geläufig (z.B. beschrieben in Dehmlow und Dehmlow, Phase Transfer Catalysis; Verlag Chemie; Weinheim 1983).

Als Lösungsmittel eignen sich insbesondere höhersiedende Kohlenwasserstoffe, niedere Alkancarbonsäuren sowie deren Ester und Amide, höhersiedende Ketone und Ether. Beispiele dafür sind Benzol, Toluol, Xylol, Dimethylformamid, Dimethylacetamid, Essigsäure, Ethylacetat, Diisopropylether, Ethylenglykoldimethylether, Tetrahydrofuran, Dioxan, 2-Butanon oder Methylpropylketon.

Das als Ausgangsverbindung verwendete 4-Methyltetrahydrophthalsäureanhydrid II ist literaturbekannt (Beilstein, Handbuch der Organ. Chemie Band 17III/IV S.6003). In besonders vorteilhafter Weise kann diese Verbindung durch säurekatalysierte Isomerisierung von 4-Methyl-1,2,3,6-tetrahydrophthalsäureanhydrid nach literaturbekannten Verfahren (Chem. Abs. 78 71559 k) hergestellt werden.

Die Aniline der Formel III sind teilweise neu. Sie können nach an sich bekannten Verfahren hergestellt werden durch:

a) Reduktion von Nitroverbindungen der Formel VI

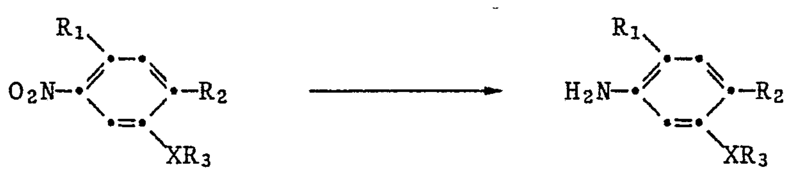

(VI) (III)

z.B. durch katalytische Verfahren (wie $H_2/Pd/C$, $H_2/Pt$ etc.) oder

b) Umsetzung eines Phenols oder Thiophenols der Formel VII mit einer Verbindung der Formel V

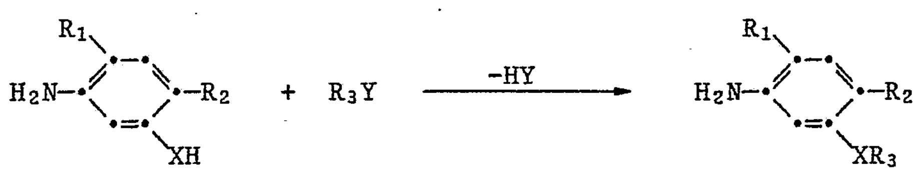


(VII) (V) (III)

gewünschtenfalls unter Basenzusatz.

In den Formeln (III), (V), (VI) und (VII) haben die Reste $R_1$, $R_2$, $R_3$ und X die für Formel I angegebene Bedeutung. Y steht für eine unter den Reaktionsbedingungen austauschbare Gruppe, wie Halogen, vorzugs weise Chlor, Brom oder Jod oder für einen gewünschtenfalls im Phenylkern alkylierten oder halogenierten Phenylsulfonylrest oder für ein Radikal der Formel $R_3$-O-$SO_2$-O.

Des weiteren betrifft die Erfindung die neuen Verbindungen der Formel IV

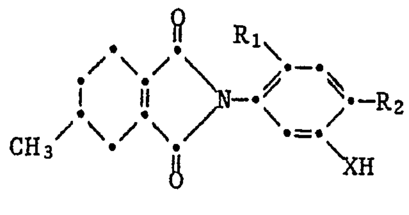 (IV),

worin
$R_1$ Wasserstoff oder Fluor
$R_2$ Halogen
X O oder S
bedeutet.

Bevorzugt sind Verbindungen der Formel IV, in der $R_2$ Fluor, Chlor oder Brom bedeutet.

Die Verbindungen der Formel IV sind erhältlich durch Umsetzung von 4-Methyl-3,4,5,6-tetrahydrophthalsäureanhydrid II mit einem Anilin der Formel VII

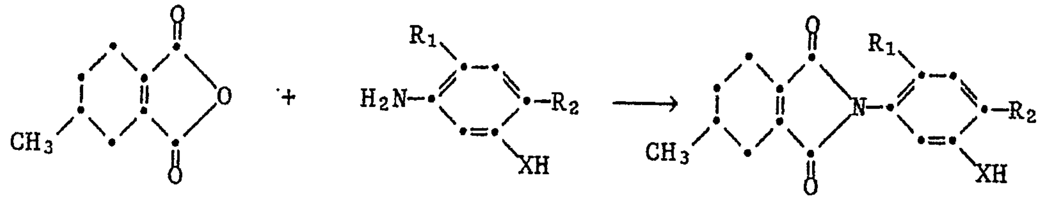

(II) (VII) (IV)

worin
$R_1$ Wasserstoff oder Fluor
$R_2$ Halogen und
X O oder S bedeutet.

Das Verfahren kann analog zu dem vorstehend beschriebenen Verfahren a) zur Herstellung von Verbindungen der Formel I durgeführt werden.

Die Verbindungen der Formel I sind hochaktive Pflanzenwirkstoffe, welche sich bei geeigneten Aufwandmengen hervorragend als Selektiv-herbizide zur Unkrautbekämpfung in Nutzpflanzenkulturen eignen. Das heisst, bei diesen Aufwandmengen zeichnen sich die Wirkstoffe der Formel I durch gute selektiv-hebizide Eigenschaft gegen Unkräuter aus. Kulturpflanzen wie Roggen, Gerste, Hafer, Weizen, Mais, Hirse, Reis, Baumwolle und Soja bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung gleichermassen mit gutem Erfolg verwendet werden.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen

$C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tension sind auch Tensidgemische zu verstehen.

Geeignete anionische Tenside können sowohl sog. wasserlösliche Seifen als auch wasser lösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionsiche Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglycol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:
"1986 International Mc. Cutcheon's Emulsifiers & Detergents" Glen Rock, N.J. USA;
H. Stache, "Tensid-Taschenbuch", 2. Auflage., C. Hanser Verlag, München, Wien, 1981;
M. and J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %

flüssige Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoff: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %

Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1

Herstellungsbeispiele

Beispiel 1a

Herstellung von N-(4-Chlor-2-fluor-5-isopropyloxyphenyl)-4-methyl-3,4,5,6-tetrahydrophthalsäureimid.

45,0 g (0.27 mol) 4-Methyl-3,4,5,6-tetrahydrophthalsäureanhydrid werden in 300 ml Toluol gelöst. Nach Zugabe von 54,2 g (0,27 mol) 2-Fluor-4-Chlor-5-isopropyloxy-anilin sowie 0,5 g Dimethylaminopyridin heizt man auf, bis das Lösungsmittel und das entstandene Wasser abdestillieren. Nach ca. 2 Std. bei 145°C Badtemperatur ist die Reaktion beendet. Der Rückstand wird mit Hexan/Esslgester 9:1 über eine Kielselgelsäure chromatographiert.

Man isoliert 75,3 g (79,3 %) der Titelverbindung der Formel

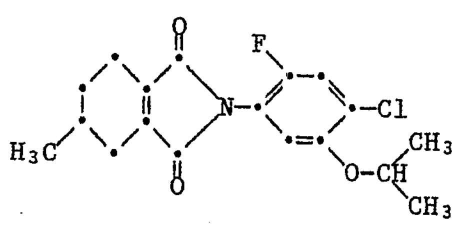

als farblose Kristalle vom Fp. = 88-89°C (Verbindung No. 1.031).

Beispiel 1b

Herstellung von N-(4-Chlor-5-ethoxy-2-fluorphenyl)-4-methyl-3,4,5,6-tetrahydrophthalsäureimid.

6,1 g (0,02 mol) N-(2-Fluor-4-chlor-5-hydroxyphenyl)-4-methyl-3,4,5,6-tetrahydrophthalsäureimid werden zusammen mit 3,0 g (0,022 mol) Kaliumcarbonat in 50 ml Methylpropylketon auf 80°C geheizt. Nach 30 Min. fällt dickes Salz aus. Bei 40°C gibt man 1,95 ml (0,024 mol) Ethyljodid zu und lässt das nun wieder dünnflüssige Reaktionsgemisch noch für 2 Std. bei 45°C ausrühren. Nach dem Abfiltrieren dampft man das Filtrat ein und kristallisiert das zurückbleibende Oel aus Ether/Hexan um.

Man isoliert 5,6 g (83,6 %) der Titelverbindung der Formel

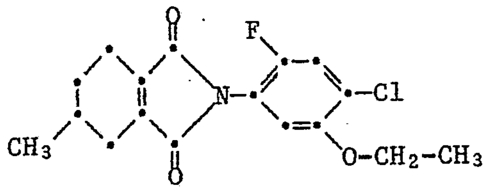

als hellbeige Kristalle vom Schmp. 108-109°C (Verbindung No. 1.029).

Analog zu Beispiel 1a und 1b sind die nachstehend genannten Verbindungen der Formel I

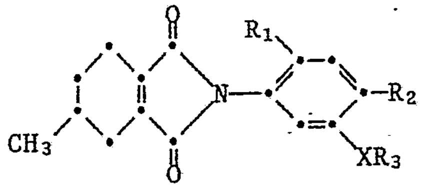

(I) ,

erhältlich.

| Verb.No. | $R_1$ | $R_2$ | X | $R_3$ | phys. Daten |
|---|---|---|---|---|---|
| 1.001 | H | Cl | O | $CH_3$ | |
| 1.002 | H | Cl | O | $C_2H_5$ | |
| 1.003 | H | Cl | O | $n-C_3H_7$ | |
| 1.004 | H | Cl | O | $i-C_3H_7$ | |
| 1.005 | H | Cl | O | $n-C_4H_9$ | |
| 1.006 | H | Cl | O | $i-C_4H_9$ | |
| 1.007 | H | Cl | O | $s-C_4H_9$ | |
| 1.008 | H | Cl | O | $t-C_4H_9$ | |
| 1.009 | H | Cl | O | $n-C_5H_{11}$ | |
| 1.010 | H | Cl | O | $i-C_5H_{11}$ | |
| 1.011 | H | Cl | O | Cyclopentyl | |
| 1.012 | H | Cl | O | Cyclohexyl | |
| 1.013 | H | Cl | O | $CH_2-CH=CH_2$ | |
| 1.014 | H | Cl | O | $CH_2-\underset{CH_3}{C}=CH_2$ | |
| 1.015 | H | Cl | O | $CH_2-CH=CH-CH_3$ | |
| 1.016 | H | Cl | O | $CH_2-C\equiv CH$ | |
| 1.017 | H | Cl | O | $CH_2-C\equiv C-CH_3$ | |
| 1.018 | H | Br | O | $CH_3$ | |
| 1.019 | H | Br | O | $C_2H_5$ | |
| 1.020 | H | Br | O | $n-C_3H_7$ | |
| 1.021 | H | Br | O | $i-C_3H_7$ | |
| 1.022 | H | Br | O | $n-C_4H_9$ | |
| 1.023 | H | Br | O | $i-C_4H_9$ | |
| 1.024 | H | Br | O | $n-C_6H_{13}$ | |
| 1.025 | H | Br | O | $CH_2-CH=CH_2$ | |
| 1.026 | H | Br | O | $CH_2-CH=CH-CH_3$ | |
| 1.027 | H | Br | O | $CH_2-C\equiv CH$ | |

| Verb.No. | $R_1$ | $R_2$ | X | $R_3$ | phys. Daten |
|---|---|---|---|---|---|
| 1.028 | F | Cl | O | $CH_3$ | Fp.=111-112°C |
| 1.029 | F | Cl | O | $C_2H_5$ | Fp.=108-109°C |
| 1.030 | F | Cl | O | $n-C_3H_7$ | |
| 1.031 | F | Cl | O | $i-C_3H_7$ | Fp.=88-89°C |
| 1.032 | F | Cl | O | $n-C_4H_9$ | $n_D^{21}$=1.3905 |
| 1.033 | F | Cl | O | $i-C_4H_9$ | Fp.=93-95°C |
| 1.034 | F | Cl | O | $s-C_4H_9$ | |
| 1.035 | F | Cl | O | $t-C_4H_9$ | |
| 1.036 | F | Cl | O | $n-C_5H_{11}$ | |
| 1.037 | F | Cl | O | $i-C_5H_{11}$ | |
| 1.038 | F | Cl | O | Cyclopentyl | |
| 1.039 | F | Cl | O | Cyclohexyl | |
| 1.040 | F | Cl | O | $n-C_6H_{13}$ | |
| 1.041 | F | Cl | O | $CH_2-CH=CH_2$ | |
| 1.042 | F | Cl | O | $CH_2-\underset{CH_3}{\underset{\mid}{C}}=CH_2$ | |
| 1.043 | F | Cl | O | $CH_2-CH=CH-CH_3$ | |
| 1.044 | F | Cl | O | $CH_2-C\equiv CH$ | |
| 1.045 | F | Cl | O | $CH_2-C\equiv C-CH_3$ | |
| 1.046 | F | Cl | O | 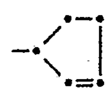 | |
| 1.047 | F | Cl | O | 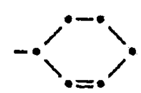 | |
| 1.048 | F | Cl | O | $CH_2-COOCH_3$ | Fp.=101-102°C |
| 1.049 | F | Br | O | $CH_3$ | |
| 1.050 | F | Br | O | $C_2H_5$ | |
| 1.051 | F | Br | O | $n-C_3H_7$ | |
| 1.052 | F | Br | O | $i-C_3H_7$ | |
| 1.053 | F | Br | O | $n-C_4H_9$ | |
| 1.054 | F | Br | O | $i-C_4H_9$ | |
| 1.055 | F | Br | O | $s-C_4H_9$ | |
| 1.056 | F | Br | O | $t-C_4H_9$ | |
| 1.057 | F | Br | O | $n-C_5H_{11}$ | |
| 1.058 | F | Br | O | $i-C_5H_{11}$ | |

| Verb.No. | $R_1$ | $R_2$ | X | $R_3$ | phys. Daten |
|---|---|---|---|---|---|
| 1.059 | F | Br | O | Cyclopentyl | |
| 1.060 | F | Br | O | Cyclohexyl | |
| 1.061 | F | Br | O | $n-C_6H_{13}$ | |
| 1.062 | F | Br | O | $CH_2-CH=CH_2$ | |
| 1.063 | F | Br | O | $CH_2-\underset{CH_3}{\underset{\vert}{C}}=CH_2$ | |
| 1.064 | F | Br | O | $CH_2-CH=CH-CH_3$ | |
| 1.065 | F | Br | O | $CH_2-C\equiv CH$ | |
| 1.066 | F | Br | O | $CH_2-C\equiv C-CH_3$ | |
| 1.067 | F | Br | O | 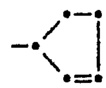 | |
| 1.068 | F | Br | O | 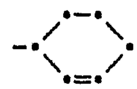 | |
| 1.069 | H | Cl | S | $CH_3$ | |
| 1.070 | H | Cl | S | $C_2H_5$ | |
| 1.071 | H | Cl | S | $n-C_3H_7$ | |
| 1.072 | H | Cl | S | $i-C_3H_7$ | |
| 1.073 | H | Cl | S | $n-C_4H_9$ | |
| 1.074 | H | Cl | S | $CH_2-CH=CH_2$ | |
| 1.075 | H | Cl | S | $CH_2-C\equiv CH$ | |
| 1.076 | H | Br | S | $CH_3$ | |
| 1.077 | H | Br | S | $C_2H_5$ | |
| 1.078 | H | Br | S | $n-C_3H_7$ | |
| 1.079 | H | Br | S | $i-C_3H_7$ | |
| 1.080 | H | Br | S | $n-C_4H_9$ | |
| 1.081 | H | Br | S | $CH_2-CH=CH_2$ | |
| 1.082 | H | Br | S | $CH_2-C\equiv CH$ | |
| 1.083 | F | Cl | S | $CH_3$ | |
| 1.084 | F | Cl | S | $C_2H_5$ | |
| 1.085 | F | Cl | S | $n-C_3H_7$ | |
| 1.086 | F | Cl | S | $i-C_3H_7$ | |
| 1.087 | F | Cl | S | $n-C_4H_9$ | |
| 1.088 | F | Cl | S | $i-C_4H_9$ | |
| 1.089 | F | Cl | S | $s-C_4H_9$ | |

| Verb.No. | $R_1$ | $R_2$ | X | $R_3$ | phys. Daten |
|---|---|---|---|---|---|
| 1.090 | F | Cl | S | $t-C_4H_9$ | |
| 1.091 | F | Cl | S | $n-C_5H_{11}$ | |
| 1.092 | F | Cl | S | Cyclopentyl | |
| 1.093 | F | Cl | S | Cyclohexyl | |
| 1.094 | F | Cl | S | $CH_2-CH=CH_2$ | |
| 1.095 | F | Cl | S | $CH_2-C(CH_3)=CH_2$ | |
| 1.096 | F | Cl | S | $CH_2-CH=CH-CH_3$ | |
| 1.097 | F | Cl | S | $CH_2-C\equiv CH$ | |
| 1.098 | F | Br | S | $CH_3$ | |
| 1.099 | F | Br | S | $C_2H_5$ | |
| 1.100 | F | Br | S | $n-C_3H_7$ | |
| 1.101 | F | Br | S | $i-C_3H_7$ | |
| 1.102 | F | Br | S | $n-C_4H_9$ | |
| 1.103 | F | Br | S | $i-C_4H_9$ | |
| 1.104 | F | Br | S | $s-C_4H_9$ | |
| 1.105 | F | Br | S | $t-C_4H_9$ | |
| 1.106 | F | Br | S | $n-C_5H_{11}$ | |
| 1.107 | F | Br | S | Cyclopentyl | |
| 1.108 | F | Br | S | Cyclohexyl | |
| 1.109 | F | Br | S | $CH_2-CH=CH_2$ | |
| 1.110 | F | Br | S | $CH_2-C(CH_3)=CH_2$ | |
| 1.111 | F | Br | S | $CH_2-CH=CH-CH_3$ | |
| 1.112 | F | Br | S | $CH_2-C\equiv CH$ | |

Beispiel 2:

Formulierungsbeispiele

Beispiel 2.1: Formulierungsbeispiele für Wirkstoffe der Formel I (% = Gewichtsprozent)

| a) Emulsionskonzentrate | a) | b) | c) |
|---|---|---|---|
| Wirkstoff gemäss Herstellungsbeispiel 1 | 20 % | 40 % | 50 % |
| Ca-Dodecylbenzolsulfonat | 5 % | 8 % | 5,8 % |
| Ricinusöl-polyethylenglykolether (36 Mol EO) | 5 % | - | - |

| | | | |
|---|---|---|---|
| Tributylphenoyl-polyethylenglykolether (30 mol EO) | – | 12 % | 4,2 % |
| Cyclohexanon | – | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Herstellungsbeispiel 1 | 80 % | 10 % | 5 % |
| Ethylenglykol-monomethylether | 20 % | – | – |
| Polyethylenglykol MG 400 | – | 70 % | – |
| N-Methyl-2-pyrrolidon | – | 20 % | 5 % |
| Epoxidiertes Kokosnussöl | – | – | 90 % |

Die Lösungen sind zur Anwendung in Form kleinster Tropfen geeignet.

| c) Granulate | a) | b) |
|---|---|---|
| Wirkstoff gemäss Herstellungsbeispiel 1 | 5 % | 10 % |
| Kaolin | 94 % | – |
| Hochdisperse Kieselsäure | 1 % | |
| Attapulgit | – | 90 % |

Der Wirkstoff wird gelöst, auf den Träger aufgesprüht und das Lösungsmittel anschliessend im Vakuum abgedampft.

| d) Stäubemittel | a) | b) | c) |
|---|---|---|---|
| Wirkstoff gemäss Herstellungsbeispiel 1 | 2 % | 5 % | 8 % |
| Hochdisperse Kieselsäure | 1 % | 5 % | 5 % |
| Talkum | 97 % | – | – |
| Kaolin | – | 90 % | 87 % |

Durch inniges Vermischen der Trägerstoffe mit dem Wirkstoff erhält man gebrauchsfertiges Stäubemittel.

e) Spritzpulver

| | a) | b) |
|---|---|---|
| Wirkstoff gemäss Herstellungsbeispiel 1 | 20 % | 60 % |
| Na-Ligninsulfonat | 5 % | 5 % |
| Na-Laurylsulfat | - | 6 % |
| Octylphenolpolyethylenglykolether (7-8 Mol EO) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

f) Extruder Granulat

| | |
|---|---|
| Wirkstoff gemäss Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

| | |
|---|---|
| Wirkstoff gemäss Herstellungsbeispiel 1 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff gemäss Herstellungsbeispiel 1 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3:

Biologische Beispiele

Beispiele 3.1: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet.

In diesem Versuch zeigen die Verbindungen nach Herstellungsbeispiel 1 starke Herbizidwirkung.

Beispiel 3.2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und dieser bei 24°-26° C und 45-60 % relativer Luftfeuchtigkeit gehalten. 15 Tage nach der Behandlung wird der Versuch ausgewertet. Auch in diesem Versuch zeigen die Verbindungen nach Herstellungsbeipiel 1 starke bis sehr starke Herbizidwirkung.

Beispiel 3.3: Selektivität gegenüber Getreide im Vorauflauf

In einer Versuchsandordung gemäss Beispiel 3.1 werden Verbindung A (gemäss Beispiel 1.031) und die aus der EP-A 61741 bekannte Verbindung B der Formeln

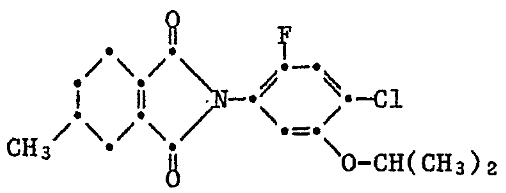

A (Verb. gemäss Beispiel 1.031)

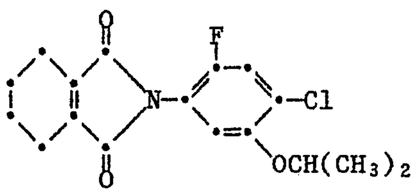

B

bei 1000, 500 und 250 g/ha Aufwandmenge miteinander verglichen.

Der Zustand der Pflanzen wird dabei gemäss folgender Bewertungsskala bonitiert:

1 Pflanze abgestorben oder hat nicht gekeimt

2-8 abnehmende Schadstufen

9 keine Schädigung, die Pflanze gedeiht wie unbehandelte Kontrollpflanzen

Man findet die nachstehend angegebenen Herbizidwirkungen (1. Spalte 1000 g/ha, 2. Spalte 500 g/ha, 3. Spalte 250 g/ha) für A und B:

| Verbindung | | A | | | B | |
|---|---|---|---|---|---|---|
| | 1. | 2. | 3. Spalte | 1. | 2. | 3. Spalte |
| Weizen | 8 | 9 | 9 | 7 | 7 | 8 |
| Gerste | 7 | 8 | 9 | 2 | 6 | 7 |
| Sinapis | 1 | 1 | 1 | 1 | 1 | 1 |
| Capsella | 1 | 1 | 1 | 1 | 1 | 1 |
| Chenopodium album | 1 | 1 | 1 | 1 | 1 | 1 |
| Chrysanthemum | 1 | 1 | 1 | 1 | 1 | 1 |
| Galium aparine | 1 | 2 | 2 | 1 | 3 | 7 |
| Papaver | 1 | 1 | 1 | 1 | 1 | 1 |
| Poa annua | 1 | 1 | 1 | 1 | 1 | 1 |
| Polygonum | 1 | 1 | 1 | 1 | 1 | 1 |
| Rumex | 1 | 1 | 1 | 1 | 1 | 1 |
| Stellaria | 1 | 1 | 1 | 1 | 1 | 1 |
| Veronica persica | 1 | 1 | 1 | 1 | 1 | 1 |
| Viola tricolor | 1 | 1 | 1 | 1 | 1 | 1 |

Beispiel 3.4: Selektivität gegenüber Getreide im Nachauflauf

In einer Versuchsanordnung gemäss Beispiel 3.2 werden Verbindung A (gemäss Beispiel 1.031) und die aus der EP-A 61741 bekannte Verbindung B der Formeln

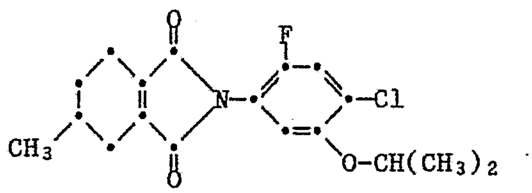

A (Verb. gemäss Beispiel 1.031)

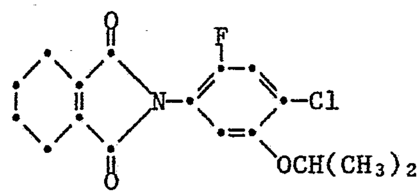

B

bei 500, 250 und 125 g/ha Aufwandmenge miteinander verglichen. Die Bonitierung wird gemäss dem in Beispiel 3.3 angegebenem Schema vorgenommen.

Man findet die nachstehend angegebenen Herbizidwirkungen (1. Spalte 500 g/ha, 2. Spalte 250 g/ha, 3. Spalte 125 g/ha) für A und B:

| Verbindung | A | | | B | | |
|---|---|---|---|---|---|---|
| | 1. | 2. | 3. Spalte | 1. | 2. | 3. Spalte |
| Weizen | 9 | 9 | 9 | 5 | 7 | 7 |
| Gerste | 9 | 9 | 9 | 5 | 6 | 6 |
| Chenopodium album | 1 | 1 | 2 | 1 | 1 | 3 |
| Chrysanthemum | 1 | 1 | 1 | 1 | 2 | 2 |
| Galium aparine | 1 | 2 | 2 | 2 | 2 | 2 |
| Papaver | 1 | 1 | 1 | 1 | 1 | 1 |
| Polygonum | 1 | 1 | 2 | 1 | 1 | 1 |
| Rumex | 1 | 1 | 1 | 1 | 1 | 2 |
| Stellaria | 2 | 2 | 2 | 3 | 4 | 5 |
| Veronica persica | 1 | 1 | 1 | 1 | 1 | 1 |
| Viola tricolor | 1 | 1 | 2 | 2 | 2 | 3 |

Beispiel 3.5 Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 0,5 bis 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die Verbindungen gemäss Herstellungsbeispiel 1 schädigen dabei die Unkräuter, nicht aber den Reis.

Beispiel 3.6: Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit Wirkstoffen gemäss Herstellungsbeispiel 1 in Aufwandmengen bis zu 300 g/ha behandelten Pflanzen eine deutliche Reduktion des Neuzuwachses, ohne dass dabei die Versuchspflanzen geschädigt wurden.

Beispiel 3.7: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6

Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken erfindungsgemässe Wirkstoffe gemäss Herstellungsbeispiel 1 eine merkliche Erhöhung der Anzahl und des Gewichts der Schoten im Haupttrieb.

Beispiel 3.8: Wuchshemmung bei Getreide

In Kuststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes gemäss Herstellungsbeispiel 1 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 3.9: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes gemäss Herstellungsbeispiel 1 besprüht. Die Wirkstoffmenge beträgt umgerechnet bis zu 500 g Aktivsubstanz pro Hektar. 21 Tage nach Applikation wird das Wachstum der Gräser beurteilt.

Verbindungen gemäss Herstellungsbeispiel 1 bewirken eine Reduzierung des Neuzuwachses im Vergleich zur unbehandelten Kontrolle.

**Patentansprüche**

1. Verbindungen der Formel I

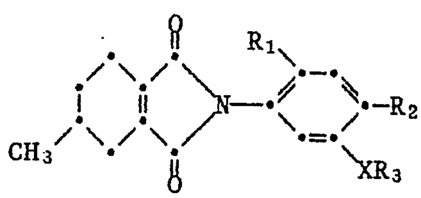

(I) ,

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet.

2. Verbindungen der Formel I nach Anspruch 1,
worin
$R_1$ Wasserstoff oder Fluor,
$R_2$ Fluor, Chlor oder Brom
X O oder S,
$R_3$ $C_3$-$C_5$-Alkenyl; $C_3$-$C_5$-Alkinyl; Cyclopentenyl; Cyclohexenyl; $C_5$-$C_6$-Cycloalkyl; oder $C_1$-$C_6$-Alkyl welchnes gewünschtenfalls einfach substituiert ist durch $C_1$-$C_4$-Alkoxy, $C_1$-$C_4$-Alkoxycarbonyl, $C_1$-$C_4$-Alkylthio, $C_1$-$C_4$-Alkylthiocarbonyl, Cyano oder ein- oder mehrfach gleich oder verschieden substituiert ist durch Fluor, Chlor oder Brom;
bedeutet.

3. Verbindungen der Formel

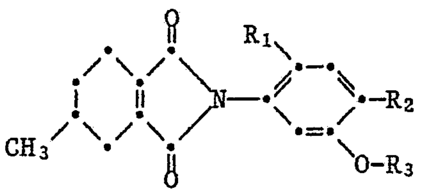

(I) ,

nach Anspruch 1 oder 2,
worin
$R_1$ Fluor
$R_2$ Chlor
$R_3$ Methyl, iso-Butyl, n-Butyl, Isopropyl oder Ethyl
bedeutet.

4. N-(4-Chlor-2-fluor-5-isopropyloxyphenyl)-4-methyl, 3,4,5,6-tetrahydrophthalimid,
N-(4-Chlor-2-fluor-5-methoxyphenyl)-4-methyl-3,4,5,6-tetrahydrophthalimid,
N-[4-Chlor-2-fluor-5-(methoxycarbonylmethyl)-phenyl]-4-methyl-3,4,5,6-tetrahydrophthalimid,
N-(4-Chlor-2-fluor-5-isobutyloxy-phenyl)-4-methyl-3,4,5,6-tetrahydrophthalimid,
N-(4-Chlor-2-fluor-5-n-butyloxy-phenyl)-4-methyl-3,4,5,6-tetrahydrophthalimid oder
N-(4-Chlor-5-ethoxy-2-fluor-phenyl)-4-methyl-3,4,5,6-tetrahydrophthalimid, gemäss Anspruch 1 oder 2.

5. Verfahren zur Herstellung von Verbindungen der Formel I gemäss Anspruch 1, dadurch gekennzeichnet, dass man
a) 4-Methyl-3,4,5,6-tetrahydrophthalsäureanhydrid der Formel II mit einem Anilin der Formel III,

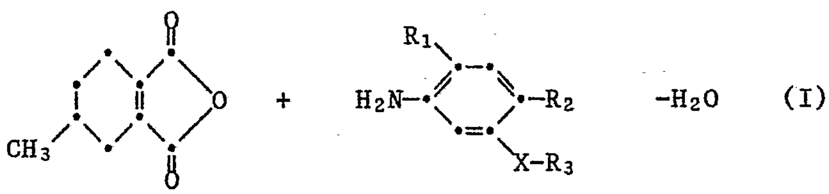


(II) (III)

worin
$R_1$ Wasserstoff oder Fluor,
$R_2$ Halogen
X O oder S,
$R_3$ $C_3$-$C_{10}$-Alkenyl; $C_5$-$C_6$-Cycloalkyl; $C_5$-$C_6$-Cycloalkenyl: $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, kondensiert oder
b) ein Phenol oder Thiophenol der Formel IV mit einer Verbindung der Formel V,

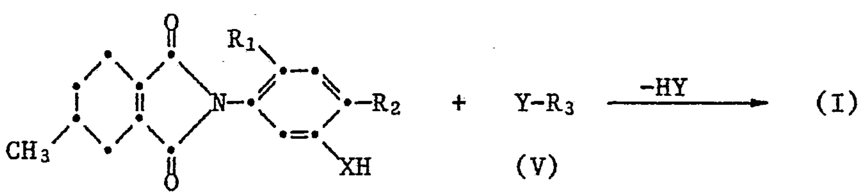


(IV) (V)

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, und
Y für Halogen, vorzugsweise Chlor, Brom oder Jod oder einen gewünschtenfalls im Phenylkern alkylierten

oder halogenierten Phenylsulfonylrest oder ein Radikal der Formel $R_3OSO_2$-O steht, umsetzt.

6. Aniline der Formel III

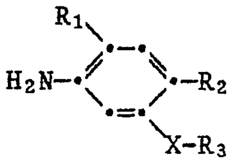

(III),

worin
$R_1$ Wasserstoff oder Fluor,
$R_2$ Halogen
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet.

7. Verfahren zur Herstellung von Anilinen der Formel III, nach Anspruch 6, dadurch gekennzeichnet dass man
a) eine Nitroverbindung der Formel VI

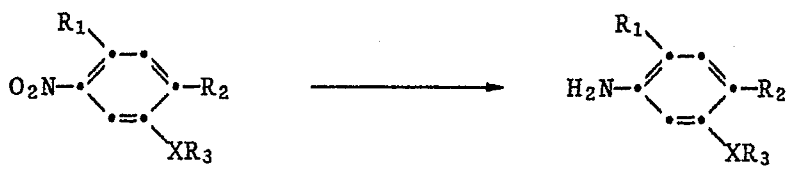

(VI) (III)

durch katalytische Verfahren reduziert oder
b) ein Phenol oder Thiphenol der Formel VII mit einer Verbindung der Formel V

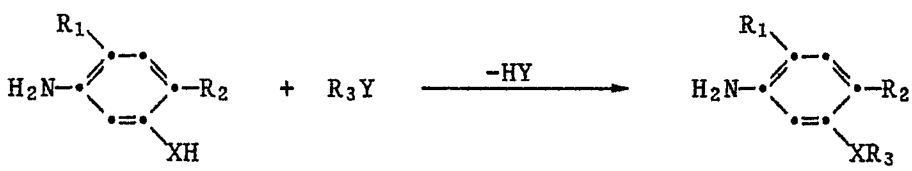


(VII) (V) (III)

in der Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, unter Basenzusatz umsetzt.

8. Verbindungen der Formel IV

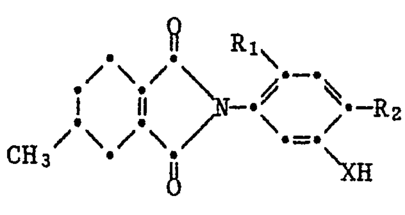

(IV),

worin
$R_1$ Wasserstoff oder Fluor
$R_2$ Halogen
X O oder S
bedeutet.

9. Verbindungen der Formel IV gemäss Anspruch 18, worin $R_2$ Fluor, Chlor oder Brom bedeutet.

10. Verfahren zur Herstellung von Verbindungen der Formel IV gemäss Anspruch 8 oder 9 durch Umsetzung von 4-Methyl-3,4,5,6-tetrahydrophthalsäureanhydrid II mit einem Anilin der Formel VII

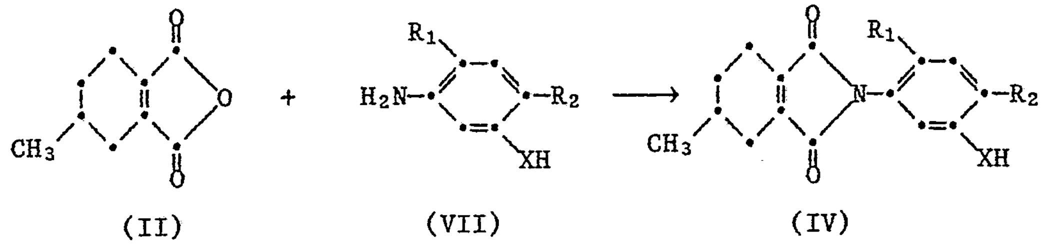

worin
$R_1$ Wasserstoff oder Fluor
$R_2$ Halogen und
X O oder S bedeutet.

11. Ein herbizides oder pflanzenwachstumsregulierendes Mittel, dadurch gekennzeichnet, dass es neben Träger- und/oder anderen Hilfsstoffen als Wirkstoff ein N-Phenyltetrahydrophthalimid der Formel I gemäss einem der Ansprüche 1 bis 4 enthält.

12. Die Verwendung eines Wirkstoffes gemäss einem der Anpsrüche 1 bis 4 oder eines einen solchen Wirkstoff enthaltenden Mittels nach Anspruch 11 zur Bekämpfung von Unkräutern.

13. Die Verwendung eines Wirkstoffes gemäss einem der Ansprüche 1 bis 4 oder eines einen solchen Wirkstoff enthaltenden Mittels nach Anspruch 11 zur Regulierung des Pflanzenwachstums.

14. Ein Verfahren zur Bekämpfung von Unkräutern, dadurch gekennzeichnet, dass man die Unkräuter oder ihren Lebensraum mit einer herbizid wirksamen Menge eines Wirkstoffes der Formel I gemäss einem der Ansprüche 1 bis 4 behandelt.

Patentansprüche für den folgenden Vertragsstaat: AT

1. Herbizides und/oder pflanzenwuchsregulatorisches Mittel enthaltend eine Verbindung der Formel I

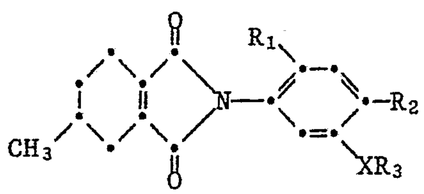

(I) ,

worin
$R_1$ Wasserstoff oder Fluor,
$R_2$ Halogen
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy; $C_1$-$C_4$-Alkoxycarbonyl, Cyano, $C_1$-$C_4$-Alkylthio oder $C_1$-$C_4$-Alkylthiocarbonyl;
bedeutet.

2. Mittel nach Anspruch 1, enthaltend eine Verbindung der Formel I, worin
$R_1$ Wasserstoff oder Fluor,
$R_2$ Fluor, Chlor oder Brom
X O oder S,
$R_3$ $C_3$-$C_5$-Alkenyl; $C_3$-$C_5$-Alkinyl; Cyclopentenyl; Cyclohexenyl; $C_5$-$C_6$-Cycloalkyl; oder $C_1$-$C_6$-Alkyl welches gewüschtenfalls einfach substituiert ist durch $C_1$-$C_4$-Alkoxy, $C_1$-$C_4$-Alkoxycarbonyl, $C_1$-$C_4$-Alkylthio, $C_1$-$C_4$-Alkylthiocarbonyl, Cyano oder ein- oder mehrfach gleich oder verschieden substituiert ist durch Fluor, Chlor oder Brom;
bedeutet.

3. Mittel nach Anspruch 1 oder 2, enthaltend eine Verbindung der Formel

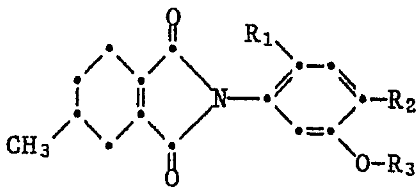

(I) ,

worin
$R_1$ Fluor
$R_2$ Chlor
$R_3$ Methyl, iso-Butyl, n-Butyl, Isopropyl oder Ethyl
bedeutet.

4. Mittel gemäss Anspruch 1 oder 2, enthaltend
N-(4-Chlor-2-fluor-5-isopropyloxyphenyl)-4-methyl-3,4,5,6-tetrahydrophthalimid,
N-(4-Chlor-2-fluor-5-methoxyphenyl)-4-methyl-3,4,5,6-tetrahydrophthalimid,
N-[4-Chloro-2-fluor-5-(methoxycarbonylmethyl)-phenyl]-4-methyl-3,4,5,6-tetrahydrophthalimid,
N-(4-Chlor-2-fluor-5-isobutyloxy-phenyl)-4-methyl-3,4,5,6-tetrahydrophthalimid,
N-(4-Chlor-2-fluor-5-n-butyloxy-phenyl)-4-methyl-3,4,5,6-tetrahydrophthalimid oder
N-(4-Chlor-5-ethoxy-2-fluor-phenyl)-4-3,4,5,6-tetrahydrophthalimid als Verbindung der Formel I.

5. Verfahren zur Herstellung von Verbindungen der Formel I gemäss Anspruch 1, dadurch gekennzeichnet, das man
a) 4-Methyl-3,4,5,6-tetrahydrophthalsäureanhydrid der Formel II mit einem Anilin der Formel III,

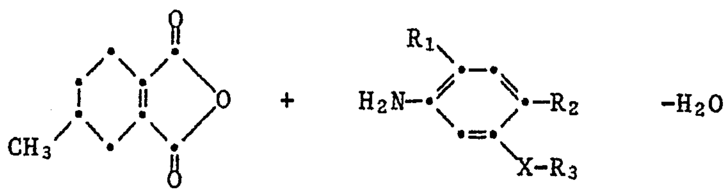


(I)

(II) (III)

worin
$R_1$ Wasserstoff oder Fluor,
$R_2$ Halogen
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewüschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, kondensiert oder
b) ein Phenol oder Thiophenol der Formel IV mit einer Verbindung der Formel V,

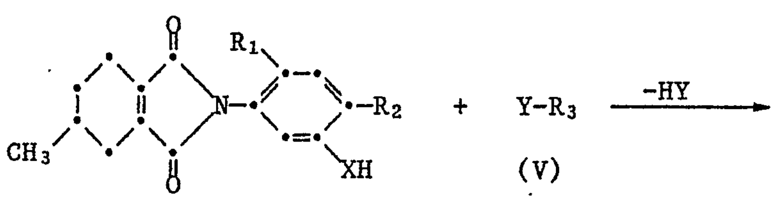


(I)

(IV) (V)

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, und
Y für Halogen, vorzugsweise Chlor, Brom oder Jod oder einen gewünschtenfalls im Phenylkern alkylierten

oder halogenierten Phenylsulfonylrest oder ein Radikal der Formel $R_3OSO_2$-O steht, umsetzt.

6. Verfahren zur Herstellung von Anilinen der Formel III

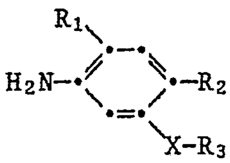

(III),

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, dadurch gekennzeichnet dass man
a) eine Nitroverbindung der Formel VI

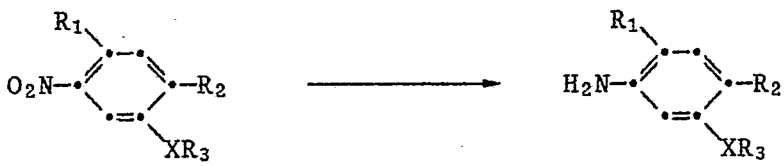

(VI) (III)

durch katalytische Verfahren reduziert oder
b) ein Phenol oder Thiophenol der Formel VII mit einer Verbindung der Formel V

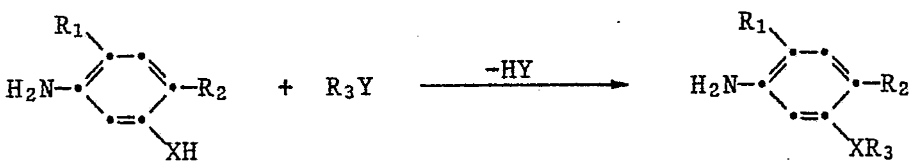


(VII) (V) (III)

in der Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, unter Basenzusatz umsetzt.

7. Verfahren zur Herstellung von Verbindungen der Formel IV

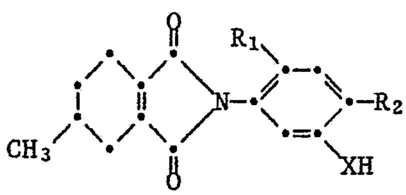

(IV),

worin
$R_1$ Wasserstoff oder Fluor
$R_2$ Halogen
X O oder S
bedeutet, dadurch gekennzeichnet, dass man 4-Methyl-3,4,5,6-tetrahydrophthalsäureanhydrid II mit einem Anilin der Formel VII,

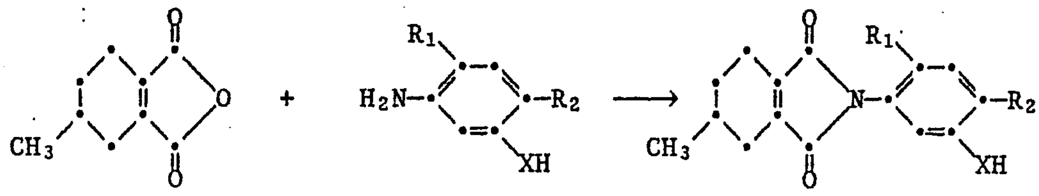

(II) (VII) (IV)

worin
$R_1$ Wasserstoff oder Fluor
$R_2$ Halogen und
X O und S bedeutet, umsetzt.

8. Die Verwendung eines Mittels gemäss einem der Ansprüche 1 bis 4 oder zur Bekämpfung von Unkräutern.

9. Die Verwendung eines Mittels gemäss einem der Ansprüche 1 bis 4 zur Regulierung des Pflanzenwachstums.

10. Ein Verfahren zur Bekämpfung von Unkräutern, dadurch gekennzeichnet, dass man die Unkräuter oder ihren Lebensraum mit einer herbizid wirksamen Menge eines Mittels gemäss einem der Ansprüche 1 bis 4 behandelt.

Patentansprüche für den folgenden Vertragsstaat: ES

1. Verfahren zur Herstellung von Verbindungen der Formel I

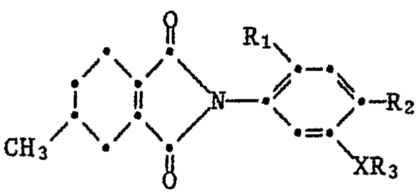

(I) ,

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, dadurch gekennzeichnet, dass man
a) 4-Methyl-3,4,5,6-tetrahydrophthalsäureanhydrid der Formel II mit einem Anilin der Formel III,

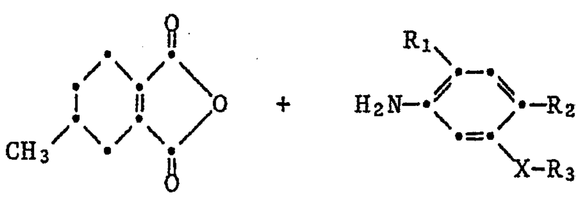

$-H_2O$ (I)

(II) (III)

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halgen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, kondensiert oder
b) ein Phenol oder Thiophenol der Formel IV mit einer Verbindung der Formel V,

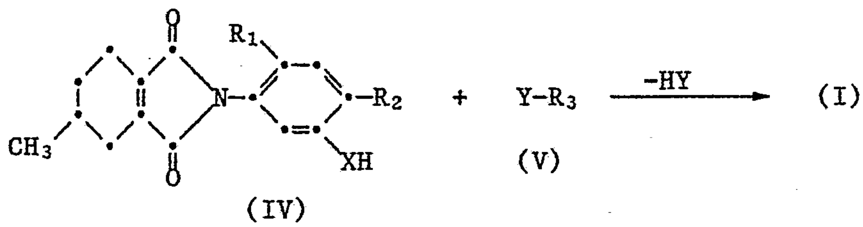

(IV)

(V)

worin
$R_1$ Wasserstoff oder Fluor,
$R_2$ Halogen,
X O oder S,
$R_3$ $C_3$-$C_{10}$-Alkenyl; $C_5$-$C_6$-Cycloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, und
Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, umsetzt.

2. Verfahren gemäss Anspruch 1, zur Herstellung von Verbindungen der Formel I
worin
$R_1$ Wasserstoff oder Fluor,
$R_2$ Fluor, Chlor oder Brom
X O oder S,
$R_3$ $C_3$-$C_5$-Alkenyl; $C_3$-$C_5$-Alkinyl; Cyclopentenyl; Cyclohexenyl; $C_5$-$C_6$-Cycloalkyl; oder $C_1$-$C_6$-Alkyl welches gewünschtenfalls einfach substituiert ist durch $C_1$-$C_4$-Alkoxy, $C_1$-$C_4$-Alkoxycarbonyl, $C_1$-$C_4$-Alkylthio, $C_1$-$C_4$-Alkylthiocarbonyl, Cyano oder ein- oder mehrfach gleich oder verschieden substituiert ist durch Fluor, Chlor oder Brom;
bedeutet.

3. Verfahren gemäss Anspruch 1, zur Herstellung von Verbindungen der Formel I
worin
$R_1$ Fluor
$R_2$ Chlor
$R_3$ Methyl, iso-Butyl, n-Butyl, Isopropyl oder Ethyl
bedeutet.

4. Verfahren nach Anspruch 1 oder 2, zur Herstellung von
N-(4-Chlor-2-fluor-5-isopropyloxyphenyl)-4-methyl-3,4,5,6-tetrahydrophthalimid,
N-(4-Chlor-2-fluor-5-methoxyphenyl)-4-methyl-3,4,5,6-tetrahydrophthalimid,
N-[4-Chlor-2-fluor-5-(methoxycarbonylmethyl)-phenyl]-4-methyl-3,4,5,6-tetrahydrophthalimid,
N-(4-Chlor-2-fluor-5-isobutyloxy-phenyl)-4-methyl-3,4,5,6-tetrahydrophthalimid,
N-(4-Chlor-2-fluor-5-n-butyloxy-phenyl)-4-methyl-3,4,5,6-tetrahydrophthalimid oder
N-(4-Chlor-5-ethoxy-2-fluor-phenyl)-4-methyl-3,4,5,6-tetrahydrophthalimid.

5. Verfahren zur Herstellung eines Anilins der Formel III

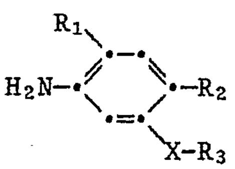

(III),

worin
$R_1$ Wasserstoff oder Fluor,
$R_2$ Halogen,
X O oder S,
$R_3$ $C_3$-$C_{10}$-Alkenyl; $C_5$-$C_6$-CYcloalkyl; $C_5$-$C_6$-Cycloalkenyl; $C_3$-$C_{10}$-Alkinyl; oder $C_1$-$C_{10}$-Alkyl welches gewünschtenfalls ein- oder mehrfach substituiert ist durch Halogen, $C_1$-$C_4$-Alkoxy, $C_1$-$C_4$-Alkoxycarbonyl, Cyano, $C_1$-$C_4$-Alkylthio oder $C_1$-$C_4$-Alkylthiocarbonyl;
bedeutet, dadurch gekennzeichnet dass man
a) eine Nitroverbindung der Formel VI

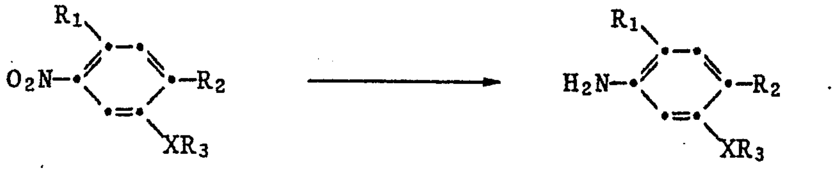

(VI) (III)

durch katalytische Verfahren reduziert oder
b) ein Phenol oder Thiophenol der Formel VII mit einer Verbindung der Formel V

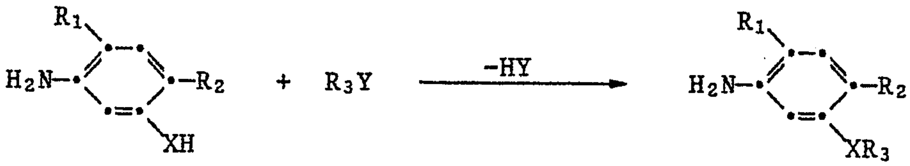


(VII) (V) (III)

in der Y für Halogen, vorzugsweise Chlor, Brom oder Jod oder einen gewünschtenfalls im Phenylkern alkylierten oder halogenierten Phenylsulfonylrest oder ein Radikal der Formel $R_3OSO_2$-O steht, unter Basenzusatz umsetzt.

6. Verfahren zur Herstellung von Verbindungen der Formel IV

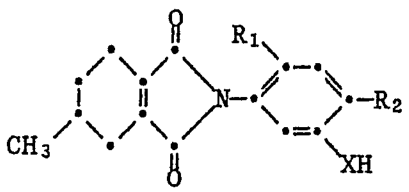

(IV),

worin
$R_1$ Wasserstoff oder Fluor
$R_2$ Halogen
X O oder S
bedeutet, gekennzeichnet durch die Umsetzung von 4-Methyl-3,4,5,6-tetrahydrophthalsäureanhydrid II mit einem Anilin der Formel VII

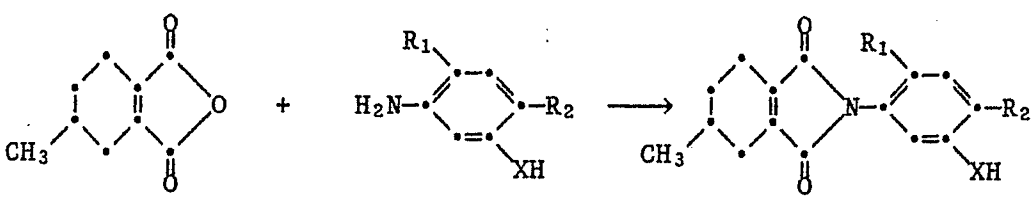

(II) (VII) (IV)

worin
$R_1$ Wasserstoff oder Fluor
$R_2$ Halogen und
X O und S bedeutet.

7. Ein Verfahren zur Bekämpfung von Unkräutern, dadurch gekennzeichnet, dass man die Unkräuter oder ihren Lebensraum mit einer herbizid wirksamen Menge eines Wirkstoffes der Formel I gemäss einem der Ansprüche 1 bis 4 behandelt.

8. Ein Verfahren zur Regulierung des Pflanzenwachstums, dadurch gekennzeichnet, dass man die Pflanzen oder deren Lebensraum mit einer pflanzenwuchsregulatorishcen Menge eines Wirkstoffes der Formel I gemäss einem der Ansprüche 1 bis 4 behandelt.

Europäisches Patentamt

# EUROPÄISCHER RECHERCHENBERICHT

Nummer der Anmeldung: EP 87810490.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | CH - A5 - 646 424 (MITSUBISHI CHEMICAL INDUSTRIES LIMITED)<br>* Zusammenfassung; Patentansprüche 11,14 *<br>-- | 1,5,11-14 | C 07 D 209/48<br>A 01 N 43/38<br>C 07 C 149/42<br>C 07 C 93/14 |
| D,A | EP - A2/A3 - 0 061 741 (SUMITOMO CHEMICAL COMPANY, LIMITED)<br>* Seite 1; Seite 3, Zeilen 8-25; Seiten 4,5; Seite 6, Zeilen 1-10; Seite 7, letzter Absatz; Seite 8, 1. Absatz; Beispiele 1-3,5,6; Tabelle 2 *<br>-- | 1,5-7, 11-14 | |
| X | PATENT ABSTRACTS OF JAPAN, unexamined applications, Field C, Band 9, Nr. 218, 5. September 1985<br>THE PATENT OFFICE JAPANESE GOVERNMENT<br>Seite 20 C 301<br>* Kokai-Nr. 60-78 959 (SUMITOMO KOGYO K.K.) *<br>-- | 6 | RECHERCHIERTE SACHGEBIETE (Int. Cl.4)<br>C 07 D 209/00<br>C 07 C 93/00<br>C 07 C 149/00 |
| A | DE - B - 1 518 404 (UNIVERSAL OIL PRODUCTS CO.)<br>* Anspruch 1 *<br>-- | 6,7 | |

Der vorliegende Recherchenbericht wurde für alle Patentansprüche erstellt.

| Recherchenort | Abschlußdatum der Recherche | Prüfer |
|---|---|---|
| WIEN | 02-12-1987 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

**EUROPÄISCHER RECHERCHENBERICHT**

Nummer der Anmeldung



EP 87810490.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | US - A - 4 292 070 (OSAMU WAKABAYASHI et al.)<br>* Tabelle 2; Spalte 4, Zeilen 22-29 *<br>-- | 6,7 | |
| X | CHEMICAL ABSTRACTS, Band 101, Nr. 17, 22. Oktober 1984, Columbus, Ohio, USA<br>SUMITOMO CHEMICAL CO., LTD. "N-phenyltetrahydrophthalamic acid derivatives"<br>Seite 685, Spalte 1, Zusammenfassung Nr. 151 490q<br>& Jpn. Kokai Tokkyo Koho JP 59 67,255 [84 67,255]<br>-- | 6 | |
| X | CHEMICAL ABSTRACTS, Band 103, Nr. 7, 19. August 1985, Columbus, Ohio, USA<br>LANGLOIS, BERNARD et al. "m-Substituted anilines"<br>Seite 624, Spalte 2, Zusammenfassung-Nr. 71 041v<br>& EP-A-0 140 783<br>-- | 6 | RECHERCHIERTE SACHGEBIETE (Int. Cl 4) |
| X | CHEMICAL ABSTRACTS, Band 70, Nr. 23, 9. Juni 1969, Columbus, Ohio, USA<br>RUYLE, WILLIAM V. et al. "Phenylbenzoic acid"<br>Seite 306, Spalte 2, Zusammenfassung-Nr. 106 209k<br>& ZA-A-67 01 021<br>-- | 6 | |

Der vorliegende Recherchenbericht wurde für alle Patentansprüche erstellt.

| Recherchenort | Abschlußdatum der Recherche | Prüfer |
|---|---|---|
| WIEN | 02-12-1987 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung



EP 87810490.0

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| X | CHEMICAL ABSTRACTS, Band 102, Nr. 5, 4. Februar 1985, Columbus, Ohio, USA<br>NAGANO, EIKI et al. "Pyridotriazolium compounds, and their use" Seite 578, Spalte 1, Zusammenfassung-Nr. 45 951b<br>& EP-A-0 116 928<br>-- | 6 | |
| X | CHEMICAL ABSTRACTS, Band 85, Nr. 25, 20. Dezember 1976, Columbus, Ohio, USA<br>NAJER, HENRY et al. "Monosubstituted piperazine derivative" Seite 560, Spalte 2, Zusammenfassung-Nr. 192 769g<br>& DE-A-2 609 574<br>-- | 6 | RECHERCHIERTE SACHGEBIETE (Int. Cl.4) |
| X | CHEMICAL ABSTRACTS, Band 103, Nr. 11, 16. September 1985, Columbus, Ohio, USA<br>SUMITOMO CHEMICAL CO., LTD. "5-Amino-4-fluoro-2-halophenylsulfide" Seite 586, Spalte 1, Zusammenfassung-Nr. 87 630x<br>& Jpn. Kokai Tokkyo Koho JP 60 78,959 [85 78,959]<br>---- | 6 | |

Der vorliegende Recherchenbericht wurde für alle Patentansprüche erstellt.

| Recherchenort | Abschlußdatum der Recherche | Prüfer |
|---|---|---|
| WIEN | 02-12-1987 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82